# EUROPEAN PATENT APPLICATION

(11) **EP 4 813 505 A1**
(43) Date of publication of application: **30.09.2026**
(21) Application number: 25382281.1
(22) Date of filing: 25.03.2025
(51) Int. Cl.: B01D 53/94, C07C 273/18, C08G 65/08

(54) **LIQUID COMPOSITION FOR UREA SOLUTIONS IN DIESEL ENGINES, PRODUCT COMPRISING THEREOF AND USE THEREOF**

(71) Applicant: Comercial Química Massó S.A., 08029 Barcelona (ES)
(72) Inventor: GARCÍA-FARIA COLL, Fernando, 08029 Barcelona (ES); GELIDA JANÉ, Marta, 08029 Barcelona (ES); TEJEL GORGAS, Rafael, 08029 Barcelona (ES); PEACE SUNDAY, Perfect, 08029 Barcelona (ES)
(74) Representative: Ponti & Partners, S.L.P

(57) **Abstract**

The present invention relates to a liquid composition for urea solutions in diesel engines, as well as a product for selective catalytic reduction (SCR) systems in diesel engines comprising said composition. The present invention also relates to the use of said composition as an additive for urea solution injection systems used in diesel engines.

## Description

### Field of the invention

The present invention relates to a liquid composition for urea solutions in diesel engines, as well as a product for selective catalytic reduction (SCR) systems in diesel engines comprising said composition. The present invention also relates to the use of said composition as an additive for urea solution injection systems used in diesel engines.

### Background of the invention

AdBlue^{®} is a 32.5% urea solution in water that was designed to comply with the stringent Euro 6 anti-pollution regulations through a catalytic reduction process of CO and NOx gases. During this process, urea (CH₄N₂O) is converted into ammonia (NH₃) at high temperatures. In contact with water and gases from engine combustion, carbon monoxide (CO) and nitrogen oxides (NOx), which are harmful to health, are eliminated.

In cars, AdBlue^{®} is injected into exhaust gases that are generally in the range of 200 to 450 °C, depending on the engine load and speed. In trucks and heavy-duty vehicles, exhaust gases are in a wider range, generally between 200 and 550 °C, but AdBlue^{®} is not injected until the exhaust gases reach at least 200-250 °C. This ensures that AdBlue^{®} decomposes thermally into ammonia (NH₃) and does not form residues in the system. This ammonia should later mix with NOₓ pollutants to become clean N₂.

If the exhaust gases do not reach a sufficient temperature, the urea in AdBlue^{®} does not break down completely into ammonia and carbon dioxide, and may instead form solid residues such as biuret, cyanuric acid and ammelides. This risk is greater on short journeys or under low engine load conditions, where the exhaust gases do not reach 200°C consistently. In addition to these three main residues, calcium carbonate may also form if hard water was used carelessly in the manufacture of AdBlue^{®}.

Modern systems are designed to prevent AdBlue^{®} injection if the exhaust gases are too cold, minimizing thereby residue formation. However, in cases where the system is not working efficiently (e.g. injector contamination or poor mixing), temperatures of 200-250 °C are critical for the formation of solid residues, especially cyanuric acid.

As can be seen, system efficiency is related to temperature, and this is related to engine design, ambient temperature, driving type and time, among other factors. Therefore, the formation of residues is directly related to this temperature and the design of the engine and the SCR (Selective Catalytic Reduction) system. Since in most cases it is not possible to make changes to the design of these systems, it is essential to use a complementary system that can interact with the residues being generated.

Ammelide, cyanuric acid and biuret, and as mentioned above, calcium carbonate, are, in descending order, highly polar compounds, but at the same time, they are very insoluble in water because the atoms composing them are bound by very intense intramolecular forces, forming structures that precipitate easily. These precipitates prevent perfect injection and can damage the SCR system. Ultimately, the purpose for which AdBlue was introduced is defeated, the engine returns to polluting the atmosphere with nitrogen oxides and incurs expensive repairs for the end consumer.

To prevent this, products have been developed to reduce the surface tension of AdBlue with the aim of reducing droplet size and thus promoting homogeneous injection. By reducing the droplet size, the formed residues should aggregate less and therefore reduce the formation of precipitates at the tip of the injector. Among all the proposed systems, the vast majority use non-ionic surfactants with a high HLB between 10 and 20 of the amine/amide type, fatty acid esters and ethoxylated and/or propoxylated alcohols. Patents dealing with this problem are described below.

Patent DE102007020281 proposes the use of a surfactant containing a hydrophilic functional group that can form a hydrogen bond. Carboxyl, carboxylate, hydroxyl, sulphone, sulphate, sulphonate, ammonium, amino, amide, polyether alcohols and betaines are indicated as possibilities.

Patent EP2129452 - USE OF AN AQUEOUS SOLUTION IN SCR SYSTEMS FOR THE TREATMENT OF DIESEL ENGINE EXHAUST GASES AND SCR METHOD proposes the use of a non-ionic alkoxylated alcohol surfactant with an HLB between 7 and 17. Along the same lines, patent EP2337625, among others, proposes a METHOD FOR MINIMISING THE DIAMETER OF DROPLETS OF A UREA SOLUTION BY USING A SURFACTANT MIXTURE OF ALKOXYLATED COMPOUNDS.

Patent CN104084086 entitled SURFACTANT FOR AUTOMOTIVE UREA SOLUTION AND METHOD FOR PREPARING AND APPLYING THE SAME proposes the use of a non-ionic surfactant of aliphatic amine polyoxypropylene polyoxyethylene ether.

Patent CN108409957, entitled FLUOROCARBON SURFACTANT FOR UREA SOLUTION FOR VEHICLES, proposes the use of a non-ionic surfactant of fluorocarbonated alkanol polyether.

Patent CN112791587, entitled UREA SOLUTION WITH HIGH ATOMISATION PROPERTIES FOR VEHICLES AND METHOD FOR PREPARING THE SAME proposes a mixture of the two previous ones by mixing surfactants based on polyoxypropylene ether and polyoxyethylene fatty amine.

Patent CN117443193, entitled ADBLUE ADDITIVE FOR VEHICLES AND METHOD FOR PREPARING THE SAME, proposes the use of polyether amines in combination with alkali metal ions.

Patent EP1140327, entitled ADDITIVE FOR THE TREATMENT OF UREA EXHAUST GASES, proposes the use of oxygenated organic compounds of the polyoxyalkylamine glycol type.

Existing solutions minimise this problem but do not eradicate it, pointing to the need for further research into more effective compounds. The above mentioned patents took droplet size reduction as a key but almost monographic aspect in the reduction of residues in the AdBlue^{®} injector and the SCR system. In response to this, the present inventors aim to achieve greater and more efficient atomisation of AdBlue^{®} with the use of new surfactant active ingredients not previously used, and we are committed to new mechanisms to improve atomisation.

The present invention, in addition to improving the atomisation of the aqueous urea solution, adds new ways to reduce the residues that are deposited in the AdBlue^{®} injectors. It also incorporates other features which, although not part of the main function, add new secondary functions that improve the operability of the mixture and injection, increase the durability of the product and open up new commercial avenues for introducing this additive to the market.

Among the features that improve the main function of the additive are:
**(A)** greater and more efficient atomisation of **AdBlue^{®},**
(B) greater chemical affinity of the additive with the chemistry of the residues,
(C) greater thermal stability of the additive, which reinforces the stability of the urea by delaying its degradation and promoting less waste generation, as it has been possible to withstand higher temperatures when the reactions expected for obtaining ammonia occur;
(D) greater ability to solubilise, disperse and emulsify residues, keeping urea residues (such as biuret, cyanuric acid and ammelides) in a stable liquid phase, whether dissolved, dispersed or forming emulsions, preventing them from precipitating or forming deposits.

Among the characteristics that are not part of the main function, we highlight the fact that the compounds mainly proposed by the market (fatty acid esters, amines/amides and ethoxylated alcohols) form a large amount of foam during the process of mixing the additive with AdBlue^{®}. This problem is important because it does not allow for easy mixing at an industrial level, requiring more time or a review of the pumping system. Once the foam is generated, it persists for a long time because it does not collapse easily and disappear.

In this regard, the present invention proposes a system with the following additional advantages:
(E) It generates much less foam than what is currently available on the market, and the little foam that is generated is reduced in a matter of seconds.
(F) greater biodegradability; and
(G) lower toxicity, promoting a greener and less aggressive solution for users.

In addition, the market today only offers a solution for adding the additive directly to the tank, whether in a car or truck. The composition of the present invention provides a new solution to the various manufacturers of 32.5% urea in water (Adblue^{®}) who were previously unable to offer this function to their customers because the market was dominated by the few multinational brands that had developed this additive for their own use. The composition of the present invention can be presented as a product for the aftermarket and for Adblue^{®} manufacturers.

Both formulations have different rationales in terms of the consumer of the additive, its dosage, the proposed ratio of active ingredients, the use of other functional additives, etc. UDROP 400-800, by using smaller doses, reduces the water content, ensuring surgical addition and reducing the impact on treatment costs and additive supply logistics. Therefore, this new proposal for the market represents (H) an economic and competitive advantage by providing access to many manufacturers and, ultimately, to the end user.

Finally, the composition of the present invention has made it possible to **(I) increase the product's life for aftermarket.** Traditionally, Adblue^{®} is a product with a high water content, which compromises its stability over time and all the problems associated therewith.

### Brief description of the invention

The present invention relates, in a first aspect, to a liquid composition for urea solutions in diesel engines. In a second aspect, the present invention relates to a product for selective catalytic reduction (SCR) systems in diesel engines comprising the composition according to the first aspect. In a third aspect, the present invention relates to the use of the composition according to the first aspect of the invention as an additive or for the manufacture of additives for urea solution injection systems used in diesel engines. In a fourth aspect, the present invention relates to a process for treating urea solutions in post-combustion catalytic filters in diesel engines comprising the addition of said composition according to the first aspect of the invention.

### Detailed description of the invention

In a first aspect, the present invention relates to a liquid composition for urea solutions, preferably Adblue^{®}, in diesel engines comprising a non-ionic surfactant of the ethylene oxide and propylene oxide triblock copolymer type with a hydrophilic/lipophilic balance (HLB) between 8 and 18.

In this disclosure and in the claims, terms such as "comprises," "comprising," "containing," and "having" are open-ended terms and may mean "includes," "including," and the like; while terms such as "consisting of" or "consists of" refer to the elements mentioned after these terms and exclude others which are not mentioned.

Unless otherwise explained, all technical and scientific terms used in this document have the same meaning as commonly understood by an expert in the technical field to which this disclosure belongs. The singular terms "a" "an," and "the" include plural references unless the context clearly indicates otherwise. For example, if "a solution" is indicated, it is understood that there may be one or more solutions. Similarly, the word "or" is intended to include "and" unless the context clearly indicates otherwise.

It should be noted that the term "about" applied to values used above and below in this document includes a margin of error of ± 5%, such as ± 4%, ± 3%, ± 2%, ± 1%.

It should be noted that in the present invention, reference will often be made to the product Adblue^{®} as it is the best known and most widely marketed product as a urea solution in diesel engines, but the present invention is equally directed to urea solutions that are not necessarily Adblue^{®}.

In a preferred embodiment, the non-ionic surfactant of the ethylene oxide and propylene oxide triblock copolymer type with a hydrophilic/lipophilic balance (HLB) between 8 and 18 inclusive in the composition of the invention is a poloxamer with at least 40 carbon atoms and a molecular weight between 2,000 and 15,000 g/mol. Preferably, the poloxamer has a maximum of 700 carbon atoms. In a more preferred embodiment, the poloxamer is selected from the group consisting of Poloxamer 338, Poloxamer 407 (Pluronic F127), Poloxamer 188 (Pluronic F68), Poloxamer 184 (Pluronic L64) and Poloxamer 181 (Pluronic L-61 - EO20-PO70-EO20).

In another preferred embodiment, the composition further comprises a non-ionic surfactant based on a glucoside with an HLB between 8 and 18. Preferably, the non-ionic surfactant based on a glucoside is an alkylglucoside with a total of 7 to 24 carbon atoms. In a more preferred embodiment, the alkylglucoside is selected from the group consisting of dodecylglucoside, decylglucoside, laurylglucoside and hexylglucoside (CAS No. 54549-24-5).

In another preferred embodiment, the composition further comprises one or more of an antifoaming compound, a preservative and ammonia. Preferably, the antifoaming compound is selected from silicones and silicone polymers. More preferably, the antifoaming compound is a silicone emulsion with a molecular weight of between 1,000 and 20,000 g/mol.

In another preferred embodiment, the antifoaming compound is present in the total composition in up to 0.5% by weight.

In another preferred embodiment, the preservative is present in the total composition in up to 0.5% by weight.

In another preferred embodiment, ammonia is present in the total composition in up to 10% by weight.

In the present invention, when the expression "a non-ionic surfactant" is used in reference to the components of "non-ionic surfactant of the ethylene oxide and propylene oxide triblock copolymer type with a hydrophilic/lipophilic balance (HLB) between 8 and 18" and "non-ionic surfactant based on glucose with an HLB between 8 and 18", it should be understood that each of these also includes the possible mixture of non-ionic surfactants that meet the indicated requirements. That is, for example, a non-ionic surfactant of the ethylene oxide and propylene oxide copolymer triblock type with a hydrophilic/lipophilic balance (HLB) between 8 and 18 may include a single type of surfactant with these characteristics or a mixture of different types with the same characteristics. The same applies to non-ionic surfactants based on glucosides with an HLB between 8 and 18.

The composition of the present invention can be obtained by simply mixing its components and there is no limitation on their quantities in the mixture except that the total of the components must add up to 100%, taking into account the possible concentrations of additional additives, if any.

Below is a comparison of the structure and classification of the active surfactants forming part of the invention, compared with the surfactants that have been proposed on the market to date:

| **Surfactant** | **Chemical structure** | **Carbon Range** | **Molecular Weight (Da)** | **Main Applications** | **Key Properties** |
|---|---|---|---|---|---|
| **PPO-PEO Blocks** (Poloxamers) | (PEO-PPO-PEO) (block copolymer) | 20-200 (variable) | 1,000-14,000 | Pharmaceutical, biotechnology, cosmetics | Gelling agents, biocompatible, modifiable |
| **Natural** (Alkyl glucosides (AG)) | Alkyl (C8-C16) + glucose | 14-22 | 300-600 | Cosmetics, eco-friendly cleaning, detergents | Mild, biodegradable, pH tolerant |
| **Ethoxylated Alcohols and phenols (EA)** | R-(OCH₂CH₂)n-OH | 14-40 | 200-1,500 | Detergents, textiles, emulsifiers | Adjustable HLB, good detergency |
| **Nitrogenated** (Ethoxylated Amines / Amides) | R-NH-(CH₂CH₂O)n-H R-CO-NH-(CH₂CH₂O)n-H | 14-40 | 300-1,500 | Agricultural products, cosmetics, disinfectants and liquid detergents | Antistatic agents, antimicrobial activity |
| **Fatty acid esters (FAE)** | R-COO-R' | 16-36 | 300-1,200 | Food, cosmetics, lubricants | Mild emulsifiers, high biodegradability |

| | | | | | |
|---|---|---|---|---|---|
| PPO: Polypropylene oxide PEO: Polyethylene oxide | | | | | |

Among all these differences, it is worth highlighting the case of poloxamers, which have longer chains, more complex structures and are often used in pharmaceutical applications.

With regard to the advantages indicated in the Background to the invention section:

### (A) Greater atomisation and greater efficiency

When we talk about better atomisation of aqueous urea solutions, or AdBlue^{®} in particular, we are talking about a smaller and more consistent droplet size, which must take into account three factors:
▪ The lower **the surface tension of the surfactant,** the lower the energy at the surface of the droplet and therefore the smaller the AdBlue^{®} droplets and, consequently, the lower the amount of residue in each droplet, which will lead to less agglomeration of the droplets and therefore to a lower amount of residue in the AdBlue^{®} injectors.
▪ The lower **the CMC (critical micelle concentration),** the less surfactant will be needed to achieve an effective reduction in surface tension. The higher the CMC, the higher the concentration needed to achieve the same effect.
▪ The greater **the interfacial stability,** the more effectively the surface tension will be reduced, decreasing the surface energy. Interfacial stability, in this case, refers to the ability of the liquid interface (AdBlue^{®}) and gas (air in the bubbles) to maintain their structure without the droplets, which are susceptible to carrying unwanted residues, joining together. This effect is achieved by reducing the coalescence of the droplets and thus preventing them from collapsing and forming larger droplets that could promote the formation of larger residue aggregates. Therefore, a greater interfacial stability promotes finer and more uniform atomisation of AdBlue^{®} when injected into the exhaust system. If the interface between the AdBlue^{®} solution and the air or exhaust gases is stable, the AdBlue^{®} droplets will be smaller and disperse better, improving NOx reduction efficiency.

This stability depends on the adsorption of the surfactant at the interface, as surfactants reduce the interfacial energy by forming a protective layer around the droplets. If the layer is strong and stable, the droplets do not merge easily, improving the stability of the system. This stability also depends on other aspects such as interfacial viscosity, steric and electrostatic interactions, and the interface residence time, which we will not discuss here.

Ethylene oxide and propylene oxide triblock copolymers, particularly poloxamers, have a hydrophobic central core of polypropylene oxide (PPO) and two hydrophilic ends of polyethylene oxide (PEO). The combination of hydrophobic and hydrophilic segments in adjustable proportions allows for better orientation at the interface, optimising their surfactant activity. This arrangement allows for more efficient adsorption at the water-air interface, forming a more stable layer at the interface, reducing surface tension more effectively and decreasing surface energy. Poloxamers also have an extremely low CMC (critical micelle concentration), which means that they can self-assemble and stabilise interfaces even at low concentrations. In summary, poloxamers are more effective at reducing surface tension due to their triblock structure, high adsorption ability, molecular flexibility and low CMC, making them more efficient than we would expect when looking solely at their surface tension. They are therefore ideal for stabilising emulsions and preventing the coalescence of AdBlue^{®} droplets.

On the other hand, alkyl glucosides reduce surface tension better than poloxamers due to their chemical structure and superior surfactant properties. One of the key reasons is their more efficient amphiphilic structure. The glucoside group has a large, highly hydrophilic polar head and a strong hydrophobic tail. Alkyl glucosides form a denser and more stable layer at the water-air interface, which reduces surface energy more effectively. This structure allows for faster and more stable adsorption at the interface, reducing surface tension more efficiently. However, the CMC is higher, making them less efficient than poloxamers, alcohols or ethoxylated amines. Nevertheless, they are biodegradable and provide good interfacial stability.

Ethoxylated alcohols, amines/amides, on the other hand, have lower surface tension, contributing to the fast initial reduction in droplet size in this regard, but they also tend to be more flexible and less compact at the interface, decreasing their ability to reduce surface tension, which can lead to less droplet coalescence over time. They have an intermediate CMC, which makes them effective at low concentrations, but not as effective as poloxamers.

Ethoxylated amides and fatty acid esters (FAEs) have higher CMC and moderate surface tension reduction. They are not the best option for improving AdBlue atomisation due to their lower efficiency compared to other alternatives.

Finally, if we consider the combination of poloxamers and alkyl glucosides, a more effective combination is obtained. On the one hand, they combine low surface tension with very low CMC, which allows the dose required to obtain an optimal effect to be reduced. In addition, they provide excellent interfacial stability, which prevents droplet coalescence. For all these reasons, they are the most efficient option in terms of atomisation and durability.

Below is a comparison of the surface tension of each surfactant to understand which one or ones will help us reduce and homogenise the droplet size.

| **Position** | **Surfactant** | **Overall Atomisation Efficiency** | **Surface Tension (mN/m)** | **CMC (g/L)** | **Interfacial Stability** |
|---|---|---|---|---|---|
| 1 | Poloxamers + Alkyl Glucosides | Maximum efficacy | 22-26 mN/m | Low (~0.05-0.2 g/L) | Excellent |
| 2 | Ethoxylated alcohols (EA) | High | 20-25 mN/m | Medium (~0.1-0.5 g/L) | Moderate |
| 3 | Ethoxylated amines | High | 21-26 mN/m | Medium (~0.1-0.5 g/L) | Moderate |
| 4 | Alkyl glucosides (AG) | Good | 24-28 mN/m | High (~0.5-5 g/L) | High |
| 5 | Poloxamers (Pluronics) | Good | 28-32 mN/m | Very low (~0.001-0.02 g/L) | Very high |
| 6 | Ethoxylated amides | Medium | 26-30 mN/m | High (~0.5-5 g/L) | Good |
| 7 | Fatty acid esters (FAE) | Medium | 25-35 mN/m | High (~0.5-5 g/L) | Moderate |

Therefore, although in the short term, ethoxylated alcohols and ethoxylated amines may be considered better options because they reduce surface tension somewhat better, poloxamers combined with alkyl glucosides are the optimal choice in the long term, as they require less surfactant (low CMC) and provide the best combination of surface tension reduction and interfacial stability.

In spray systems such as AdBlue^{®}, interfacial stability is crucial because it determines whether the formed droplets remain small or begin to coalesce in the air or on the engine surface. Although surfactants such as ethoxylated alcohols rapidly reduce surface tension, the interfacial stability obtained is not sufficient and the droplets coalesce again. This characteristic is achieved more effectively with the use of poloxamers because they form a much more stable interface, which prevents small droplets from coalescing again.

### (B) Greater chemical affinity of the additive with the chemistry of the residues

The main residues that can form deposits in AdBlue^{®} injectors have highly polar structures rich in nitrogen and oxygen functional groups.

| **Residue** | **Main Chemical Structure** | **Key Properties** |
|---|---|---|
| **Biuret** | NH₂-CO-NH-CO-NH₂ | **Highly polar, hydrophilic, forms hydrogen bonds** |
| **Cyanuric acid** | C₃H₃N₃O₃ | **Polar, aromatic, with π-π interactions and hydrogen bonds** |
| **Ammelides** | Derivatives of cyanuric acid | **Polar, with electrostatic and hydrogen interactions** |

The affinity of the surfactant with AdBlue^{®} and its residues (such as cyanuric acid, biuret and ammelides) is a key factor when it comes to reducing deposit formation in AdBlue^{®} injectors. The way a surfactant interacts with these components affects its ability to prevent crystallisation or the formation of insoluble residues during the injection process in selective catalytic reduction (SCR) systems.

This affinity of the surfactant with the residues depends on its ability to interact with nitrogen compounds and to form stable micelles that prevent crystallisation and deposition in the injectors. This affinity is determined by the following factors:
**Chemical interactions between the residues and the surfactant.** The affinity of AdBlue ^{®} residues will depend on how well the chemical structure of the surfactant matches that of the residues. Surfactants can capture and stabilise these residues through three main mechanisms:
- *Hydrogen bonds* (strong hydrophilic interactions) These occur when there is a hydrogen donor group (NH, OH) and a hydrogen acceptor group (C=O, N, O) in the surfactant molecule. Alkyl glucosides and poloxamers have -OH groups that can form hydrogen bonds with AdBlue^{®} residues. Therefore, surfactants capable of forming hydrogen bonds will have a high affinity for biuret and cyanuric acid.
- *Electrostatic interactions.* Partially charged surfactants can stabilise residues with opposite charges. Ethoxylated amines can interact with anionic residues due to the presence of partially positively charged nitrogen. Effective with cyanuric acid, but less so with biuret.
- *Solubilisation in micelles.* Surfactants with low CMC can form micelles that encapsulate residues, preventing their precipitation. Poloxamers and alkyl glucosides form stable micelles, which help prevent the formation of deposits by dispersing residues.

| **Surfactant** | **Hydrophilic group** | **Hydrophobic group** | **Type of Interaction with Residues** | **Affinity with Residues** |
|---|---|---|---|---|
| **Poloxamers** | Polyethylene glycol (PEO) | Polypropylene glycol (PPO) | Hydrogen bonds + micelles | **High** |
| **Alkyl glucosides** | Sugar (glucose) | Alkyl chain | Hydrogen bonds | **High** |
| **Ethoxylated amines** | Amine group + EO | Alkyl chain | Electrostatic interactions | **Medium-high** |
| **Ethoxylated alcohols** | OH group + EO | Alkyl chain | Solubilisation in water | **Medium** |
| **Ethoxylated Amides** | Amide group + EO | Alkyl chain | Low solubilisation | **Low** |
| **Fatty acid esters** | Ester group | Long chain | Weak interactions | **Very low** |

**Solubilisation ability of polar compounds.** AdBlue^{®} residues are theoretically soluble in water because, like water, they are highly polar. However, strong internal interactions in the structure of these residues prevent their solubility in water and promote the formation of aggregates that can easily crystallise and form deposits. To prevent these deposits, surfactants must surround them and keep them in solution, preventing them from agglomerating and precipitating. The most effective surfactants are those that can interact with these polar molecules through hydrogen bonds, electrostatic interactions or solubilisation in micelles.

**CMC (Critical Micelle Concentration):** the lower the CMC, the greater the effectiveness at low concentrations. This point has already been discussed above.

**Interfacial stability:** A greater interfacial stability promotes finer and more uniform atomisation of AdBlue^{®} when injected into the exhaust system. This point has already been discussed above.

Surfactants with high affinity for residues can prevent their formation by keeping them dispersed in the AdBlue^{®} solution, thus preventing their precipitation. In this case, both urea and cyanuric, biuret and ammelide residues are highly polar. Therefore, it is advisable that surfactants also be highly hydrophilic. These surfactants can also dissolve residues, preventing them from depositing. If a surfactant can form a protective film at the interfaces, this can prevent urea residues from accumulating on the surfaces of the injectors, maintaining the stability of the AdBlue solution.

Poloxamers are highly polar. In addition, they have a triblock structure (hydrophobic-hydrophilic-hydrophobic) that allows them to adsorb efficiently at interfaces and form micelles. Therefore, they have a relatively good affinity for AdBlue^{®} residues due to their ability to interact with both hydrophilic components (urea, water) and more hydrophobic residues (biuret, cyanuric acid) because of the stability of their structures, not their polarity. Therefore, their ability to form micelles and the flexibility of their structure make them good at dissolving residues and preventing deposits.

Alkyl glucosides are also highly polar and have an amphiphilic structure (a polar head and a non-polar tail) that allows them to adsorb well on surfaces and keep residues dispersed. Their affinity for the hydrophobic residues in AdBlue^{®}, such as biuret and cyanuric acid, is higher than that of other surfactants such as ethoxylated alcohols and ethoxylated amines.

Ethoxylated alcohols are non-ionic and have ethoxylated groups (-CH₂CH₂O), which gives them a high affinity for aqueous systems such as AdBlue^{®}. Although they help reduce surface tension, their affinity for residues such as biuret and cyanuric acid is not as high as that of alkyl glucosides or poloxamers. For this reason, they are less effective at keeping residues dispersed compared to other surfactants.

Ethoxylated amines/amides are also non-ionic surfactants, but they have a moderate affinity for AdBlue^{®} residues. Although the ethoxylated (hydrophilic) groups help to solubilise residues, amines/amides do not have the same ability to interact effectively with organic residues (such as biuret or cyanuric acid) as alkyl glucosides.

Fatty acid esters (such as ethylene glycol or PEG glycerol esters) are also non-ionic surfactants, but their ability to solubilise residues such as biuret and cyanuric acid is quite limited. Although they can reduce surface tension in the system, their affinity for hydrophobic residues is lower than that of poloxamers or alkyl glucosides. They are not as effective in preventing deposits in AdBlue injectors.

| **Position** | **Surfactant** | **Affinity for residues** | **Polarity** | **CMC (g/L)** | **Mechanism of action** |
|---|---|---|---|---|---|
| **1** | **Poloxamers + Alkyl glucosides** | **Very high** | **High** (Amphiphilic) | **Low (~0.05-0.2)** | Micelles + Hydrophilic interaction |
| **2** | **Alkyl glucosides (AG)** | **High** | **High** (Polar) | **High (~0.5-5)** | Hydrophilic solubilisation |
| **3** | **Poloxamers (Pluronics)** | **High** | **Medium-high** (amphiphilic) | **Very low (~0.001-0.02)** | Crystal stabilisation |
| **4** | **Ethoxylated Amines** | **Medium-high** | **Medium** (Moderately polar) | **Medium (~0.1-0.5)** | Electrostatic interaction |
| **5** | **Ethoxylated alcohols (EA)** | **Medium** | **Medium** (polar) | **Medium (~0.1-0.5)** | Particle dispersion |
| **6** | **Ethoxylated amides** | **Low-Medium** | **Low-medium** (slightly polar) | **High (~0.5-5)** | Low solubilisation |
| **7** | **Fatty acid esters (FAE)** | **Low** | **Low** (non-polar) | **High (~0.5-**5) | Low affinity with residues |

Therefore, the combination of poloxamers and alkyl glucosides has the best affinity with residues. Their high polarity, structure compatible with nitrogenous residues, high interfacial stability to prevent deposits, offered by poloxamers, together with a low CMC that allows micelles to form even at low concentrations and the ability of alkyl glucosides to solubilise residues.

### (C) Greater thermal stability

The thermal stability of surfactants plays a crucial role in protecting urea from thermal degradation during the injection process in SCR (selective catalytic reduction) systems and prevents the formation of undesirable residues such as cyanuric acid, biuret and ammelides. These compounds can form when urea decomposes at high temperatures, which can clog or damage AdBlue^{®} injectors and the SCR system, as already mentioned.

Factors affecting the thermal stability of surfactants include bond energy, molecular structure rigidity and the stability of functional groups in the hydrophilic part.

Long chains of saturated hydrocarbons (such as in poloxamers and alkyl glucosides) are more thermally stable because they have strong C-C and C-H bonds (around 350-410 kJ/mol). They generally have high molecular weights and rigid structures due to their multiple branches. If the chain is very long and flexible, it can degrade by thermal cracking, but in the temperature range of AdBlue^{®} systems, this is not the main problem. Short chains can be more reactive due to greater molecular mobility, facilitating thermal reactivity. In addition, short chains have lower conformational stability, which increases the likelihood of degradation by oxidation or thermal reactions.

Furthermore, the type of functional groups in the hydrophilic part also has an effect due to the nature of the chemical bonds within the molecule. The following table shows the type of breakdown that can occur in different surfactants. **Esters and amides** are less thermally stable than **ethoxylated alcohols, alkyl glucosides and poloxamers.**

| **Functional Group** | **Type of Weak Bond** | **Thermal Breakdown Mechanism** | **Approximate Decomposition Temperature** |
|---|---|---|---|
| **Esters (R-COO-R')** | Ester bond (C=O-O) | **Thermal hydrolysis** → Breaks down into acid and alcohol | **100-150°C** (especially in aqueous media) |
| **Amides (R-CO-NH₂)** | Amide bond (C=O-NH) | **Thermal hydrolysis** → Breaks down into acid and amine | **150-250°C** |
| **Ethoxylated alcohols (-OH + EO)** | No direct labile bond | May oxidise, but resists heat well | **200-300°C** |

| **Functional Group** | **Type of Weak Bond** | **Thermal Breakdown Mechanism** | **Approximate Decomposition Temperature** |
|---|---|---|---|
| **Alkyl glucosides (C-O-C in ring)** | Stable glycoside bond | Requires high energy to break | **>250°C** |
| **Poloxamers (PEO-PPO-PEO)** | Strong C-C, C-O bonds | Thermally very stable | **>300°C** |

Therefore, surfactants with high thermal stability act as urea protectors, reducing the degradation rate by keeping the solution more stable at high temperatures, while interfering with the formation of undesired degradation products.

When surfactants are stable at high temperatures, they can disperse degradation products (such as biuret and cyanuric acid) and prevent them from accumulating or precipitating on the surfaces of the injector, which prevents clogging and deposit formation. Furthermore, as we have seen before, surfactants with good affinity for residues can keep them dispersed and prevent them from re-aggregating with the urea.

Poloxamers are known for their high thermal stability and heat resistance. Their triblock structure (hydrophobic-hydrophilic-hydrophobic) allows them to maintain their functionality at high temperatures, keeping urea stable and preventing thermal degradation. They therefore protect urea in the SCR system from the formation of residues such as cyanuric acid and biuret.

Alkyl glucosides have a good thermal stability (although slightly lower than poloxamers) and are highly effective in dispersing urea residues such as biuret and cyanuric acid. Although they may be slightly more sensitive to high temperatures, they remain quite effective in keeping urea stable in injection systems.

Ethoxylated alcohols have moderate thermal stability. At high temperatures (200-300°C), they may begin to degrade or lose their surfactant properties. This results in deposit formation and further degradation of urea.

Ethoxylated amines/amides also have medium to low thermal stability. Although they are effective in reducing surface tension, their ability to protect urea from thermal degradation is limited.

Fatty acid esters such as ethylene glycol have low thermal stability. At high temperatures, fatty acid esters lose their surfactant properties and degrade, causing accelerated degradation of urea.

| **Surfactant** | **Thermal stability** | **Protection against urea degradation** | **Justification** |
|---|---|---|---|
| **Poloxamers** | **Very high** | **High** | PEO-PPO-PEO block structure without labile bonds. High thermal stability. |
| | | | Reduces biuret and cyanuric acid crystallisation. |
| **Alkyl glucosides** | **High** | **High** | Resistant glycoside bonds. Strong interaction with urea through hydrogen bonds, reducing biuret formation. |
| **Poloxamer + Alkyl glucoside** | **Very high** | **Very high** | Combination of thermal stability and deposit prevention. It prevents urea degradation and disperses decomposition products. |
| **Ethoxylated amines** | **Medium-high** | **Medium** | More stable than esters and amides. They can form electrostatic interactions with urea, but are less effective than poloxamers or alkyl glucosides. |
| **Ethoxylated alcohols** | **Medium** | **Medium** | Heat resistant, but may oxidise with oxygen at high temperatures. They do not prevent biuret formation so efficiently. |
| **Ethoxylated Amides** | **Low-Medium** | **Low** | Amide bond susceptible to thermal hydrolysis. Low urea stabilisation ability. |
| **Fatty acid esters** | **Low** | **Low** | Esters break down easily at moderate temperatures. They do not stabilise urea or prevent biuret formation. |

Poloxamers and alkyl glucosides are the most effective at protecting urea from thermal degradation and dispersing residues (biuret, cyanuric acid, amides). Their high thermal stability maintains the integrity of urea during the injection process, preventing the formation of deposits. Ethoxylated alcohols, ethoxylated amines/amides and fatty acid esters, on the other hand, have moderate to low thermal stability and are therefore less effective in protecting urea and dispersing residues at high temperatures.

### (D) Greater ability to solubilise, disperse and emulsify residues

The three concepts (solubility, ability to disperse residues and ability to emulsify them) are distinct but related, as we shall see.

Solubility refers to the ability of a surfactant to dissolve a substance (in this case, urea residues) in a solution. In a system such as AdBlue^{®}, a surfactant must be able to dissolve the residues formed (such as biuret or cyanuric acid) to prevent crystallisation or precipitation.

Dispersion involves the homogeneous distribution of residues in a liquid phase, i.e. the residues do not agglomerate or form lumps or deposits, but remain finely distributed in the solution. This helps to maintain a constant flow and prevents obstructions in injection systems.

Emulsification forms a stable mixture of two immiscible liquids, such as water and oil. Surfactants can form emulsions between urea residues (which tend to be somewhat hydrophobic) and water, creating microdroplets of the residues dispersed in the aqueous medium, preventing their precipitation or the formation of crystals that could clog the injectors.

All concepts focus on keeping urea residues (such as biuret, cyanuric acid and ammelides) in a stable liquid phase, whether dissolved, dispersed or forming emulsions, preventing them from precipitating or forming deposits.

Below, we describe the factors that affect solubilisation, dispersion and emulsification ability and provide a comparative table with each of the surfactants we studied:
- **Size and structure of the surfactant:** Larger surfactants with more hydrophobic chains tend to be more effective in solubilising and dispersing insoluble residues.
- **Nature of the hydrophilic head:** Surfactants with strong hydrophilic heads tend to better solubilise polar substances, such as urea-derived residues.
- **Interactions with residues:** Some surfactants have functional groups (such as amines, esters, etc.) that can interact directly with residues to help them dissolve or disperse.

| **Surfactant** | **Solubilisation ability** | **Dispersion ability** | **Emulsification ability** | **Explanation** |
|---|---|---|---|---|
| **Poloxamers** | **High** | **Very high** | **High** | The PEO-PPO-PEO structure provides excellent solubilisation of both polar and non-polar residues and disperses them effectively. |
| **Alkyl glucosides** | **Very high** | **Very High** | **Medium-High** | The glycoside bonds are highly effective in solubilising urea-derived residues. They also have an excellent ability to disperse residues and prevent crystallisation. |
| **Poloxamer + Alkyl glucoside** | **Very High** | **Very high** | **High** | The combination enhances solubilisation and dispersion abilities, improving the stability of residues in solution. |
| **Ethoxylated Amines** | **Medium-High** | **Medium-High** | **Medium** | Moderate interactions with residues and good dispersibility, but not as effective as alkyl glucosides and poloxamers. |
| **Ethoxylated alcohols** | **Medium** | **Medium** | **Medium** | Less effective in solubilising residues and dispersion compared to alkyl glucosides and poloxamers. |
| **Ethoxylated amides** | **Low-medium** | **Low-medium** | **Low** | Solubilisation ability is more limited due to the amide structure, and they do not disperse residues effectively. |
| **Fatty Acid Esters** | **Low** | **Low** | **Low** | They do not have an effective solubilisation or dispersion ability for residues due to the nature of their ester bonds. |

Poloxamers have a high solubilising ability thanks to the formation of micelles and interaction with hydrophobic and polar compounds. They are particularly good at dispersing residues due to their block structure and stability at high temperatures. Emulsion formation is also very efficient due to their ability to handle immiscible liquids.

Alkyl glucosides are very effective in both solubilising and dispersing residues. Their glucoside structure allows for very effective interaction with urea residues, causing biuret and cyanuric acid to remain well dispersed in solution and not form crystals. Their emulsifying ability is also effective, although slightly lower than that of poloxamers.

The combination of both surfactants maximises both solubilisation and dispersion ability. Poloxamers provide thermal stability and the ability to disperse residues efficiently, while alkyl glucosides help in the interaction and solubilisation of polar residues. This combination is very powerful, especially for maintaining residue dispersion and preventing crystallisation.

Ethoxylated amines moderately solubilise urea residues due to their amine group, which has some affinity for polar compounds. However, they are not as effective as alkyl glucosides at dispersing residues. They are less effective than the previous two in general, but could still help in some systems.

Ethoxylated alcohols have a moderate solubilising and dispersing ability, but are less effective than poloxamers and alkyl glucosides. In systems with complex residues such as AdBlue, their ability to prevent residue crystallisation is limited.

Ethoxylated amides and fatty acid esters are less effective due to the chemical structure of the amide and ester bonds, which do not favour so much the solubilisation and dispersion of complex residues. In addition, their ability to handle residues such as biuret or cyanuric acid is low.

The ability of surfactants to solubilise, disperse and form emulsions with residues generated by the thermal degradation of urea is crucial to prevent the formation of deposits in AdBlue injectors. If a surfactant cannot keep residues (such as biuret or cyanuric acid) dispersed or dissolved, these residues precipitate and form blockages or deposits that affect the performance of the SCR system.

In summary, alkyl glucosides have a greater ability to solubilise and disperse residues compared to other surfactants, outperforming ethoxylated alcohols, ethoxylated amines and ethoxylated amides. Poloxamers, although very effective in solubilisation and dispersion, are slightly less effective in some specific cases compared to alkyl glucosides. The combination of poloxamer and alkyl glucoside is therefore a very good option for improving the stability of urea residues and preventing the formation of deposits.

### (E) Less foaming

Foaming is an important factor in choosing a surfactant for AdBlue^{®}, especially since foam can interfere with the mixing process of urea with water and the surfactant. It can also affect the performance of the injection system and reduce the effectiveness of the solution.

If foam is generated, it can block the injectors in the SCR (selective catalytic reduction) system, preventing AdBlue^{®} from being injected efficiently. If too much air is trapped in the form of foam, the solution will not be distributed evenly throughout the exhaust system, which will undoubtedly affect the NOx reduction process.

Excessive foam formation can make AdBlue^{®} difficult to be handled and stored. Storage tanks and pumps may experience problems with excessive foam generated during filling or circulation of the solution.

Foam can also interfere with the stability of the urea-water mixture. If foam accumulates, it can affect the homogeneous dispersion of urea and residues (such as biuret and cyanuric acid), which could result in the precipitation or aggregation of unwanted residues.

Foam accumulation can generate additional pressure in storage and transport systems, which can damage system components or even cause pump or pipe failures.

Below, we analyse the foaming behaviour of the different surfactants we are evaluating:

| **Surfactant** | **Foam Prevention Ability** | **Explanation** |
|---|---|---|
| **Poloxamers** | **Very low** | They have a hydrophobic structure (PPO) that promotes foam reduction, along with a balance of hydrophilic/hydrophobic properties. This allows them to act at the interface without forming too much foam. |
| **Poloxamer + Alkyl Glucoside** | **Low** | Combination of poloxamer (with low foaming ability) and alkyl glucoside (also with anti-foaming properties). |
| **Alkyl glucosides** | **Low** | Alkyl glucosides have a relatively simple structure, making them more stable and less prone to foaming. The presence of the glucoside group, in particular, helps to reduce foaming. |
| **Ethoxylated amines** | **Medium** | They have a mixture of hydrophilic and hydrophobic properties that promote foaming, although less so than ethoxylated alcohols. |
| **Ethoxylated alcohols** | **Medium-High** | Ethoxylated alcohols, being relatively simple structures, tend to foam more easily. They have a strong adsorption ability at interfaces, which helps to form and stabilise foam. |
| **Ethoxylated Amides** | **High** | Ethoxylated amides have longer chains that promote foam formation. The amide structure stabilises the bubbles. |
| **Fatty acid esters** | **Very high** | Due to their long and relatively simple structure, fatty acid esters are very effective at forming foam, especially in concentrated solutions. |

Poloxamers are very good at reducing foam. Their triblock structure allows them to significantly prevent foam formation. This is an advantage in the application of AdBlue^{®}, where the risk of foam clogging is an important factor.

Alkyl glucosides also generate little foam compared to other surfactants. They are suitable for AdBlue^{®} injection systems, since their tendency to foam is moderate and can be easily controlled in suitable formulations.

Ethoxylated alcohols have medium to high foaming properties, which can be a drawback in systems where stable, bubble-free solutions are required.

Ethoxylated amines/amides form foam similarly to ethoxylated alcohols, which can interfere with the flow and uniform distribution of AdBlue^{®}. This could cause operational problems, especially at high temperatures.

Therefore, the best surfactants to prevent foaming when mixed with AdBlue^{®} are poloxamers and alkyl glucosides.

Below are the foaming results of two additives based on the use of ethoxylated alcohols and the results of two additives based on poloxamers/alkyl glucosides:

| **Formulas** | **Volume of formed foam (mL)** | **Volume of foam remaining after 15 min (mL)** |
|---|---|---|
| **Formula 1 with Ethoxylated alcohols** | 350 | > 3 min. 210 ml of foam remains |
| **Formula 2 with Ethoxylated alcohols** | 200 ml | In 36 seconds, 20 ml of foam remains 2 min 30 sec. disappears |
| **Formula 3 with Poloxamer / Alkyl glucoside** | 150 ml | 0 ml (30 sec) |
| **Formula 4 with Poloxamer / Alkyl glucoside** | 100 ml | 0 ml (18 sec) |

### Note on coalescence

As indicated above, coalescence of AdBlue^{®} droplets is undesirable. Indeed, for the atomisation of the solution, it is desirable to prevent AdBlue^{®} droplets from coalescing, as this would compromise fine atomisation. Therefore, interfacial stability must be high to prevent droplet coalescence, which helps to keep them small and dispersed, ensuring good atomisation. Furthermore, the coalescence of foam generated during the handling or injection of AdBlue^{®} is undesirable. If this occurs, it is desirable for the foam to collapse quickly.

Therefore, the coalescence of AdBlue^{®} droplets is detrimental to atomisation (as it reduces efficiency), while foam coalescence is beneficial because it helps the foam tocollapse more quickly and does not interfere with the system. The use of poloxamer and alkyl glucoside achieves both effects due to the high liquid-liquid interfacial stability between the AdBlue^{®} droplets and the high liquid-gas coalescence between the AdBlue^{®} droplets and the air bubbles.

### (F) Increased biodegradability

Biodegradability does not reduce deposit formation during AdBlue^{®} injection. However, in a system such as SCR, designed to protect the environment, it would be paradoxical not to consider this aspect. Below is a comparison of the different surfactants being evaluated:

| **Surfactant** | **Biodegradability** | **Comment** |
|---|---|---|
| **Poloxamers** | High | **Poloxamers** are generally **biodegradable** and have **low** environmental **toxicity.** However, their biodegradability depends on the **specific composition** of the polymer. In general, low molecular weight **poloxamers** are more readily biodegradable. |
| **Alkyl glucosides** | Very high | **Alkyl glucosides** have **exceptional biodegradability,** as they are based on **natural sugars** (glucose) and **fatty acids.** They are known for being **eco-friendly, low** in **toxicity,** and break down quickly in the environment without forming toxic residues. |
| **Ethoxylated alcohols** | Moderate to high | The **biodegradability of ethoxylated alcohols** varies depending on the **number of ethoxyl units** (or the size of the ethoxy group). In general, low molecular weight surfactants have a higher biodegradability, but high molecular weight ones may be more resistant to biodegradation. Even so, many ethoxylated alcohols are **moderately biodegradable.** |
| **Ethoxylated Amines** | Medium-High | **Ethoxylated amines** are partially biodegradable. Although their chemical structure is less complex than that of other surfactants, the amine groups can make biodegradation slower and dependent on specific environmental factors. |
| **Ethoxylated Amides** | Medium | **Ethoxylated amides** have moderate biodegradability, as the amide groups may be less biodegradable due to their chemical stability. While some can break down, their biodegradation rate is generally slower compared to other simpler surfactants. |
| **Fatty acid esters** | Low | **Fatty acid esters** are less biodegradable than other surfactants due to their ester structure, which can be more resistant to decomposition. In addition, their biodegradation can be affected by the length of the fatty acid chain and the type of ester. |

As can be seen in the table, alkyl glucosides and poloxamers are the most biodegradable and safest option.

### (G) Lower toxicity

The toxicity of surfactants is a critical aspect of their use, especially in applications such as AdBlue^{®}, which is used in automotive SCR systems. The toxicity of a surfactant affects the safety of operators and end users who handle the product, as well as long-term environmental health. Below is a comparison of the different surfactants as studied:

| **Surfactant** | **Toxicity** | **Comments** |
|---|---|---|
| **Poloxamers** | Low | **Poloxamers** are **known for their low toxicity** to both **humans** and the **environment.** They are **biocompatible,** non-irritating, and have **low environmental toxicity.** They are widely used in industrial and cosmetic applications, making them a safe choice for **AdBlue^{®}.** |
| **Alkyl glucosides** | Very low | **Alkyl glucosides** have **low toxicity** due to their **natural origin** (sugars and fatty acids). They are **non-toxic** to humans and have a **low impact** on the environment. They are known for their **ecological safety** and **rapid biodegradability,** making them ideal for **AdBlue^{®}** and other eco-friendly products. |
| **Ethoxylated alcohols** | Low to moderate | The **toxicity** of **ethoxylated alcohols** varies depending on the **length of the ethoxylated chain.** In general, they have **low toxicity,** but if the ethoxylated chain is long, skin and eye irritation may increase. At high concentrations, they can **cause irritation** and have an **environmental impact** if they do not biodegrade quickly. |
| **Ethoxylated Amines** | Medium | **Ethoxylated amines** have moderate toxicity due to the nature of their amine groups, which can be irritating or cause allergic reactions in some people. Their use at high concentrations or in improperly formulated products can increase their toxicity. |
| **Ethoxylated Amides** | Medium-High | **Ethoxylated amides** are more toxic than other surfactants due to the presence of the amide group, which can be irritating to the skin and eyes. In some cases, the formation of by-products during manufacture can increase their toxicity. |
| **Fatty acid esters** | Moderate | **Fatty acid esters** have a **higher toxicity** compared to other surfactants due to their chemical structure, which can be more reactive and cause irritation. Their toxicity varies depending on the fatty acid chain and the purity of the product. |

In terms of toxicity and environmental safety, alkyl glucosides are clearly the most environmentally friendly and safe option for use in products such as AdBlue^{®}, closely followed by poloxamers.

Therefore, on all the points discussed, poloxamers and alkyl glucosides prove to be superior to other surfactants currently on the market, as shown in the table below:

| **Property** | **Poloxamers** | **Alkyl glucosides** | **Ethoxylated alcohols** | **Ethoxylated amines/amides** | **Ether** |
|---|---|---|---|---|---|
| **Improved atomisation** (surface tension, CMC, interfacial stability) | Very high | Very high | Moderate | Moderate | Moderate |
| **Dispersion ability** | Good | Excellent | Moderate | Moderate | Good |
| **Emulsification ability** | High | Excellent | Moderate to high | Moderate to high | High |
| **Thermal stability** | Good | Good | Moderate | Fair | Good |
| **Foaming** | Low | Low | High | High | High |
| **Affinity for residues (cyanuric acid, biuret, ammelides)** | Very high | Very High | Moderate | Moderate | Low |
| **Environmental Impact (Accumulation)** | Low | Very low | Moderate | High | Moderate |
| **Biodegradability** | Moderate | Very high | Moderate | Low | Moderate to low |
| **Toxicity (Human Health)** | Low | Very low | Moderate | Moderate to High | Moderate |
| **Toxicity (Environment)** | Low | Very low | Moderate | High | Moderate |
| **Use in AdBlue** | Very suitable | Excellent | Acceptable | Acceptable | Acceptable |

In a second aspect, the present invention relates to a product for selective catalytic reduction (SCR) systems in diesel engines comprising the composition, according to any of the embodiments of the present invention indicated above and below, and a urea solution or solutions. In a preferred embodiment, said urea solution is Adblue^{®}.

In a third aspect, the present invention relates to the use of the composition, according to any of the embodiments of the present invention indicated above and below, as an additive or for the manufacture of additives for urea solution injection systems used in diesel engines.

In a fourth aspect, the present invention relates to a process for treating urea solutions in post-combustion catalytic filters in diesel engines, comprising adding to said urea solutions, preferably Adblue^{®}, a composition according to any of the embodiments of the present invention indicated above and below.

It should be noted that any of the embodiments described in this document may be considered alone or in combination with any other embodiment described in this document, unless the context specifies otherwise. In other words, for example, a preferred option of a defined feature may be combined with a more or less preferred option of another defined feature.

The present invention will now be illustrated by examples which are in no way intended to limit the invention.

### EXAMPLES

### Materials

The following poloxamers were used to carry out the experimental tests:
**Poloxamer 338**
   - **Molecular weight:** ~14,600 g/mol
   - **Composition:** ~141 EO - 44 PO - 141 EO
   - **Number of carbon atoms:** ~696 carbon atoms
**Poloxamer 407 (Pluronic F127)**
   - **Molecular weight:** ~12,600 g/mol
   - **Composition:** ~101 EO - 56 PO - 101 EO
   - **Number of carbon atoms:** ~572 carbon atoms
**Poloxamer 188 (Pluronic F68)**
   - **Molecular weight:** ~8,400 g/mol
   - **Composition:** ~80 EO - 27 PO - 80 EO
   - **Number of carbon atoms:** ~401 carbon atoms
**Poloxamer 184 (Pluronic L64)**
   - **Molecular weight (Mw):** ~2,900 g/mol
   - **Composition:** (EO): ~20 EO - 21 PO - 20 EO
   - **Number of carbon atoms:** ~143 carbon atoms
**Poloxamer 181 (Pluronic L-61** - **EO20-PO70-EO20):**
   - **Number of carbon atoms:** 200.
   - **Approximate molecular weight:** 2,500 g/mol.

Dodecylglucoside, decylglucoside, laurylglucoside and hexylglucoside (CAS No. 54549-24-5) were used as alkyl glucosides. The various examples are shown below:

### Tests:

### Example 1. Maximum Reduction of Surface Tension

A concentrated mixture of 80% alkyl glucoside and 20% poloxamer is proposed. The main benefit is to obtain better atomisation of AdBlue^{®}, promoting evaporation and reducing deposits in the injectors. The high concentration of alkyl glucoside reduces surface tension to minimum values, improving the atomisation of AdBlue^{®} in the combustion chamber and promoting its evaporation before impact on the exhaust walls. This formula is ideal for reducing droplet size and improving the efficiency of the atomisation process in the SCR system.

### Example 2. Increased Dispersing Ability

A concentrated mixture of 40% alkyl glucoside and 60% poloxamer is proposed. The main benefit is to minimise the accumulation of residues and deposits by improving the dispersion of particles in AdBlue. The balanced ratio of poloxamer and alkyl glucoside improves the dispersion of microparticles present in AdBlue, preventing the formation of fouling in the injector and on the surface of the catalyst. It is ideal for reducing deposit formation in injectors and maintaining the cleanliness of the SCR system.

### Example 3. Improved Emulsifying Stability

A concentrated mixture of 60% alkyl glucoside and 40% poloxamer is proposed. The main benefit is that it promotes the emulsification of contaminants and residues in AdBlue, reducing fouling. The ratio between the two components balances the reduction of surface tension with the ability to stabilise emulsions, improving the dispersion of liquid impurities and combustion residues within the SCR system. It is ideal for systems where the presence of liquid contaminants in AdBlue can cause instability in the injector.

### Example 4. Optimised solubilisation

A concentrated mixture of 20% alkyl glucoside and 80% poloxamer is proposed. The main benefit is that it promotes the solubilisation of impurities in AdBlue, preventing the crystallisation of urea residues. The high presence of poloxamer improves the solubilisation of organic residues and undesired particles, reducing the possibility of crystal formation that could obstruct the ducts of the SCR system. It is ideal for preventing the crystallisation of residues and improving the compatibility of AdBlue with other additives.

### Example 5. Foam Reduction and Increased Thermal Resistance

A concentrated mixture of 9% alkyl glucoside and 91% poloxamer is proposed. The main benefit is improved thermal stability in SCR systems operating at high temperatures. The high concentration of poloxamer provides greater thermal resistance, minimising the degradation of AdBlue^{®} under extreme conditions and reducing foam formation in the injector. It is ideal for industrial applications or heavy-duty vehicles where AdBlue^{®} operates at higher temperatures.

### Example 5. Foam Reduction and Increased Thermal Resistance

A concentrated mixture with 100% poloxamer is proposed. The main benefit is an improved thermal stability in SCR systems operating at high temperatures. The high concentration of poloxamer provides greater thermal resistance, minimising AdBlue degradation under extreme conditions and reducing foam formation in the injector. It is ideal for industrial applications or heavy-duty vehicles where AdBlue^{®} operates at higher temperatures.

### Example 6. Foam Reduction and Increased Thermal Resistance

A concentrated mixture is proposed with 80% poloxamer + water and the rest of the functional additives (ammonia, antifoaming agent, antimicrobial agent). It keeps the same benefits as example 5 but requires a higher concentration of urea in the aqueous mixture.

### Example 7. Foam Reduction and Greater Thermal Resistance

A concentrated mixture is proposed with 60% poloxamer + water and the rest being functional additives (ammonia, antifoaming agent, antimicrobial agent). It keeps the same benefits as examples 5 and 6 but requires a higher concentration of urea in the aqueous mixture.

Below are several sample formulas for a formulation to prevent the crystallisation of AdBlue^{®} residues, but now aimed at the end user, commonly referred to as the *"Aftermarket*". The percentages indicated for each component are percentages by weight, and the amount of each will be such that the total sum of the entire formulation is always 100%.

| **Ingredient** | **Formula 1 (100%)** | **Formula 2 (100%)** | **Formula 3 (100%)** | **Main advantages** |
|---|---|---|---|---|
| **Poloxamer** | 5-7 % | 4-6 % | 8-15 % | **Better atomisation and thermal stability.** |
| | | | | Poloxamers improve emulsion, reduce surface tension and resist high temperatures. |
| **Alkyl glucoside** | 3-5 % | 4-6 % | ---- | **Efficient solubilisation and dispersion of residues.** Alkyl glucosides are biodegradable, disperse organic residues and reduce foam. |
| **Anti-foaming agent** | 0.1-0.2 % | 0.2-0.3 % | 0.1-0.2% % | **Foam control.** |
| | | | | Reduces foam formation and improves atomisation efficiency without compromising dispersion and emulsion properties. |
| **Biocide** | 0.05-0.1 % | 0.02-0.05 % | 0.05-0.1 % | **Prevents microbial growth.** It helps to keep product stability and prevents the accumulation of organic residues. |
| **Ammonia** | 0.5-2 % | 0.5-2 % | 0.5-2 % | **pH adjustment.** |
| | | | | Ammonia is used to regulate pH and optimise the operating conditions of the formula, especially in residues treatment. |
| **Water** | 85-90 % | 85 % | 85-90 % | **Dissolution and dilution.** Water is the main solvent, providing the base for the formulation and dissolving the active components. |
| **Advantages** | Efficient atomisation, low foaming, excellent thermal stability and biodegradability | High solubilisation and dispersion of residues, moderate foam control, good thermal stability | More effective emulsification and dispersion, more powerful foam control, better long-term thermal stability | |

### Main advantages:

1. **Formula 1:** Ideal for applications where **greater thermal stability** and **better atomisation** are **required,** with **low foam control.** Perfect for moderate temperature environments where dispersion efficiency is key.
2. **Formula 2:** Designed to provide a **balance** between **residue solubilisation, dispersion** and **foam control,** while maintaining **good thermal stability.** Ideal for applications with organic residues in moderate temperature conditions.
3. **Formula 3:** A **more robust formula** for **effective emulsification, dispersion** and **foam control in extreme thermal conditions.** Perfect for applications where long-term stability and organic residue control are essential.

## Claims

1. A liquid composition for urea solutions in diesel engines, comprising a non-ionic surfactant of the ethylene oxide and propylene oxide triblock copolymer type with a hydrophilic/lipophilic balance (HLB) between 8 and 18.

2. Composition according to claim 1, wherein the non-ionic surfactant of the ethylene oxide and propylene oxide triblock copolymer type is a poloxamer with at least 40 carbon atoms and a molecular weight between 2,000 and 15,000 g/mol.

3. Composition according to any of the preceding claims, further comprising a non-ionic surfactant based on a glucoside with an HLB between 8 and 18.

4. Composition according to claim 3, wherein the non-ionic surfactant based on a glucoside is an alkyl glucoside with between 7 and 24 carbon atoms in total.

5. Composition according to any of claims 1 to 4, further comprising one or more antifoaming compounds, a preservative and ammonia.

6. Composition according to claim 5, wherein the antifoaming compound is selected from silicones and silicone polymers.

7. Composition according to claim 6, wherein the antifoaming compound is a silicone emulsion with a molecular weight between 1,000 and 20,000 g/mol.

8. Composition according to any of claims 5 to 7, wherein the antifoaming compound is present in the total composition in up to 0.5% by weight.

9. Composition according to any of claims 4 to 8, wherein the preservative is present in the total composition in up to 0.5% by weight.

10. Composition according to any of claims 4 to 9, wherein ammonia is present in the total composition in up to 10% by weight.

11. A product for selective catalytic reduction (SCR) systems in diesel engines comprising the composition according to any of claims 1 to 10, and a solution or solutions of urea.

12. Use of the composition according to any of claims 1 to 10 as an additive or for the manufacture of additives for urea solution injection systems used in diesel engines.

13. A process for treating urea solutions in post-combustion catalytic filters in diesel engines, comprising adding to said urea solutions a composition according to any of claims 1 to 10.

14. A product according to claim 11, use according to claim 12, or process according to claim 13, wherein the urea solution is Adblue^{®}.
